# EUROPÄISCHE PATENTANMELDUNG

(11) **EP 2 193 776 A2**
(43) Veröffentlichungstag der Anmeldung: **09.06.2010**
(21) Anmeldenummer: 10002672.3
(22) Anmeldetag: 14.07.2008
(51) Int. Cl.: A61K 6/027, A61K 6/083

(54) **Dentalkomposite mit niedriger Schrumpfspannung und hoher Biegefestigkeit**

(30) Priorität: 20.07.2007 DE 102007034457
(62) Teilanmeldung aus: 08012655.0
(71) Anmelder: Heraeus Kulzer GmbH, 63450 Hanau (DE)
(72) Erfinder: Utterodt, Andreas, Dr., 61267 Neu-Anspach (DE); Ruppert, Klaus, Dr., 63477 Maintal (DE); Schaub, Matthias, Dr., 63477 Linsengericht (DE); Diefenbach, Christine, 65599 Dornburg (DE); Reischl, Kurt, 35788 Merenberg (DE); Hohmann, Alfred, 61389 Schmitten (DE); Eck, Michael, 61389 Schmitten (DE); Schönhof, Nelli, 35619 Braunfels (DE); Schneider, Jutta, 65594 Runkel (DE); Grundler, Andreas, Dr., 41542 Dormagen (DE)
(74) Vertreter: Kühn, Hans-Christian

(57) **Zusammenfassung**

Dental-Kompositmaterialien auf (Meth)acrylatbasis, wie in Anspruch 1 beschrieben, weisen einen Anteil an TCD-Monomeren in der Gesamtzusammensetzung von 10-15 Gew.% auf, und der Quotient Biegefestigkeit/Schrumpfspannung beträgt mindestens 50.

## Beschreibung

Die Erfindung betrifft Dental-Kompositmaterialien mit geringer Schrumpfspannung und hoher Biegefestigkeit.

Lichthärtende Werkstoffe auf Acrylat-/Methacrylatbasis erfahren bei radikalischer Polymerisation aufgrund des sich bei der Polymerisation reduzierenden Molekülabstandes und der damit einhergehenden Dichteerhöhung einen Volumenschrumpf. Dieser kann durch Zugabe von anorganischen Füllstoffen wie z.B. Dentalgläsern oder pyrogenen Kieselsäuren deutlich reduziert werden, da sich ein reduzierter Monomeranteil pro Volumeneinheit ergibt und die Füllstoffe während der Polymerisation nicht schrumpfen.

Bei Dentalanwendungen ist der Volumenschrumpf von großer klinischer Bedeutung, da durch die Materialschrumpfung Zugkräfte auf die Kavitätenwand übertragen werden. Bei Überschreitung einer Maximalkraft kann diese Schrumpfkraft im Extremfall zur Ablösung von der Kavitätenwand führen. In den dadurch entstandenen Randspalt können Bakterien eindringen und in der Folge Sekundärkaries entstehen.

Gemäß DE102005021332A1 wurden bereits lichthärtende Werkstoffe auf Acrylat-/Methacrylatbasis vorgestellt, die eine reduzierte Schrumpfkraft aufweisen. Dies wird durch verschiedene Maßnahmen erreicht: Nicht-agglomerierte Nanofüller, ein Füllstoffgemisch aus grob- und feinteiligen Dentalgläsern, überwiegende Substitution des hochschrumpfenden Verdünners TEDMA durch UDMA (Urethandimethacrylat), Verwendung von Tricyclodecan-Derivaten (im Folgenden abgekürzt TCD) sowie optional die Reduktion der Initiatormenge. Beispielhaft belegt ist dort nur eine Zusammensetzung, und die enthält kein TCD.

Ein Kompositmaterial wird durch innige Vermischung folgender Komponenten hergestellt aus

| | | | |
|---|---|---|---|
| Füllstoffe: | Nicht-agglomerierte Nanopartikel | 6 | Gew. Teile |
| | Dentalglas 1 µm (silanisiert) | 24 | Gew. Teile |
| | Dentalglas 8 µm (silanisiert) | 53 | Gew. Teile |
| Monomere: | Bis-GMA (Bowen) | 11 | Gew. Teile |
| | UDMA | 4 | Gew. Teile |
| | TEDMA | 2 | Gew. Teile |
| Initiator(en): | Campherchinon | 0,1 | Gew. Teile |
| Summe | | 100,1 | Gew. Teile |

Aufgabe der vorliegenden Erfindung ist die Bereitstellung eines Kompositmaterials für Dentalan-wendungen mit geringer Schrumpfkraft und hoher Biegefestigkeit. Insbesondere soll der Quotient Biegefestigkeit/Schrumpfspannung optimiert werden.

Es hat sich gezeigt, dass die in DE102005021332A1 vorgeschlagenen Materialien wesentlich verbessert werden können. Überraschenderweise wurde gefunden, dass das Verhältnis Biegefestigkeit zu Schrumpfspannung gesteigert werden kann, wenn ein Anteil an TCD Monomeren vorhanden ist, von über 10 Gew.%.

Die Erfindung betrifft somit
Dental-Kompositmaterialien mit einem Gesamtfüllstoffgehalt von 70 bis 95 Gew.% enthaltend
A) in der Füllstoffkomponente 0,5 bis 10 Gew.% nicht-agglomerierte Nanofüller mit Partikelgrößen von 1 bis 50 nm;
B) in der Füllstoffkomponente mindestens 60 Gew.% eines Füllstoffgemischs aus 50 bis 90 % grob- und 10 bis 50 % feinteiligen Dentalgläsern, welche ein Größenverhältnis, bezogen auf die mittlere Partikelgröße (d₅₀-Wert), von feinteilig zu grobteilig von 1:4 bis 1:30 aufweisen,
   i. in der als Monomermischung ein Mitglied der Gruppe TCD-di-HEMA oder TCD-di-HEA.
   ii. in der als Monomermischung 10 bis 18 % UDMA bezogen auf die Monomermischung,
   iii. in der Monomermischung Rest TEDMA und/oder multifunktionelle Vernetzer
D) bis 1 % Initiator(en) und
E) optional in der Füllstoffkomponente mindestens ein weiteres Dentalglas mit sich von den grob- und feinteiligen Dentalgläsern unterscheidender Partikelgröße, wobei der Anteil an TCD-Monomeren in der Gesamtzusammensetzung 10-15 Gew.% beträgt, und der Quotient Biegefestigkeit/Schrumpfspannung >/= 50 beträgt.

Nicht agglomerierte Nanofüller sind an sich bekannt und z.B. in WO 0130305 A1 oder am Beispiel von SiO₂ in DE 196 17 931 A1 beschrieben. Sie gehören erfindungsgemäß vorzugsweise der Gruppe bestehend aus: SiO₂, ZrO₂, TiO₂, Al₂O₃ sowie Mischungen aus mindestens zwei dieser Stoffe an.

Sie können - wie in DE 196 17 931 A1 beschrieben - in organischen Lösungsmitteln dispergiert sein, aber auch in Wasser oder Wasser enthaltenden Lösungsmittelmischungen.

Als Dentalgläser eignen sich besonders Bariumglaspulver und/oder Strontiumglaspulver. Die mittlere Partikelgröße der grobteiligen Dentalgläser beträgt vorzugsweise 5-10 µm, insbesondere um 7 µm und die der feinteiligen 0,5 bis 2 µm, insbesondere 1 µm. Optional vorhandene weitere Dentalgläser haben z.B. mittlere Korngrößen von 2-5 oder 10-50 µm.

Die Füllstoffkomponente kann demnach Dentalgläser mit insgesamt drei oder mehr Kornfraktionen aufweisen. Sie kann auch weitere, herkömmliche, auf dem Dentalgebiet übliche Füllstoffe enthalten, wie etwa Quarz-, Glaskeramik- oder Mischungen davon. Darüber hinaus können die Komposite Füllstoffe zur Erzielung einer erhöhten Röntgenopazität enthalten. Die mittlere Partikelgröße des röntgenopaken Füllstoffs liegt vorzugsweise im Bereich von 100 bis 300 nm, insbesondere 180 bis 300 nm. Als röntgenopake Füllstoffe eignen sich z.B. die in der DE 35 02 594 A1 beschriebenen Fluoride der Seltenen Erdmetalle, d. h. die Trifluoride der Elemente 57 bis 71. Ein besonders bevorzugt verwendeter Füllstoff ist Ytterbiumfluorid, insbesondere Ytterbiumtrifluorid mit einer mittleren Partikelgröße von etwa 300 nm. Die Menge des röntgenopaken Füllstoffs beträgt vorzugsweise 10 bis 50 Gew.-%, besonders bevorzugt 20 bis 30 Gew.-%, bezogen auf den Gesamtfüllstoffgehalt.

Außerdem können gefällte Mischoxide, wie beispielsweise ZrO₂/SiO₂, als Füllstoffe eingesetzt werden. Bevorzugt sind Mischoxide mit einer Partikelgröße von 200 bis 300 nm und insbesondere etwa 200 nm. Die Mischoxidpartikel sind vorzugsweise kugelförmig und weisen eine einheitliche Größe auf. Die Mischoxide haben vorzugsweise einen Brechungsindex von 1,52 bis 1,55. Gefällte Mischoxide werden vorzugsweise in Mengen 25 bis 75 Gew. % und besonders 40 bis 75 Gew.% verwendet.

Die Füllstoffe sind zur Verbesserung der Haftung zwischen Füllstoff und organischer Matrix bevorzugt silanisiert. Als Haftvermittler eignet sich besonders alpha - Methacryloxypropyltrimethoxysilan. Die Menge des eingesetzten Haftvermittlers richtet sich nach der Art und BET-Oberfläche des Füllstoffs.

Als multifunktionelle Vernetzer kommen außer TEDMA und UDMA in Frage: Diethylenglycol-di(meth)acrylat, Decandioldi(meth)acrylat, Trimethylolpropantri(meth)acrylat, Pentaerythrittetra(meth)acrylat sowie Butandioldi(meth)acrylat, 1,10-Decandioldi(meth)acrylat, 1,12-Dode-candioldi(meth)acrylat.

Zur Initiierung der Polymerisation enthalten die Komposite einen Polymerisationsinitiator, beispielsweise einen Initiator für die radikalische Polymerisation. Je nach Art des verwendeten Initiators können die Mischungen kalt, durch Licht oder heiß polymerisierbar sein.

Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch alpha, alpha '-Azo-bis(isobutyroethylester), Benzpinakol und 2,2'-Dimethylbenzpinakol sind geeignet.

Als Photoinitatoren kommen z.B. Benzoinalkylether oder -ester, Benzilmonoketale, Acylphosphinoxide oder aliphatische und aromatische 1.2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heißhärtung eingesetzt werden.

Als Initiatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet.

Es können auch dual härtende Systeme verwendet werden, z. B. Photoinitiatoren mit Aminen und Peroxiden.

Die Initiatoren werden vorzugsweise in Mengen von 0,01 bis 1 Gew.% bezogen auf die Gesamtmasse der Mischung verwendet.

Bei der Kaltpolymerisation kann es zweckmäßig sein, wenn das Kompositmaterial aufgeteilt in zwei Komponenten vorliegt, die zur Aushärtung durch Vermischen vorgesehen sind. Es ist auch möglich, das Material so bereitzustellen, dass es sowohl durch Licht als auch durch Vermischen zweier Komponenten zu härten ist.

Erfndungsgemäße Kompositmaterialien zeigen als Dentalmaterialien bevorzugt einen Quotienten Biegefestigkeit/Schrumpfspannung von >/= 35, bevorzugt >/= 40, besonders bevorzugt >/=50.

Die folgenden Beispiele erläutern die Erfindung näher. Teile- und Prozentangaben beziehen sich wie in der übrigen Beschreibung auf das Gewicht, sofern nicht anders angegeben.

### Beispiele:

Die Messergebnisse (Tabelle II) für die in der folgenden Tabelle I aufgeführten Mischungen 312 (Vergleich, mit wenig TCD), 307 (erfindungsgemäß), 349 (erfindungsgemäß), 357 (Vergleich, mit wenig TCD), und 363 (Vergleich, optimiert ohne TCD) zeigen, dass mit steigendem TCD Gehalt der Quotient Biegefestigkeit/Schrumpfspannung ansteigt. TCD Anteile über 10% zeigen Werte über 50.

**Tabelle I Rezepturen**

| Komponenten | Rezeptur **363** | Rezeptur **312** | Rezeptur **357** | Rezeptur **349** | Rezeptur **307** |
|---|---|---|---|---|---|
| | | | | | |
| UDMA | 4,03 | 4,02 | 4,03 | 2,00 | 3,92 |
| Bis-GMA | 10,76 | 9,15 | 9,15 | | |
| TEGDMA | 1,11 | 1,11 | 1,11 | 2,66 | 0,95 |
| TCDDIHEA | | 1,61 | 1,61 | 10,50 | 12,35 |
| Mutifunktionales Urethanmonomer | 0,50 | 0,50 | 0,50 | 0,50 | 1,18 |
| BHT | 0,04 | 0,04 | 0,04 | 0,04 | 0,04 |
| Nano SiO₂ (Dispersion) | 4,00 | 4,00 | 4,00 | 4,00 | 4,8 |
| Bariumaluminosilikatglasfüller 0.85 µ silanisiert | 39,50 | 15,80 | 39,50 | 40,00 | 50,28 |
| Bariumaluminosilikatglasfüller 2 µ silanisiert | | 23,70 | | | 7,66 |
| Bariumaluminosilikatglasfüller 5 µ silanisiert | 39,50 | 39,50 | 39,50 | 40,00 | 18,53 |
| Licht-Stabilisator 1 | 0,09 | 0,09 | 0,09 | 0,05 | 0,07 |
| Licht-Stabilisator 2 | 0,26 | 0,26 | 0,26 | 0,13 | 0,02 |
| DL Campherchinon | 0,03 | 0,03 | 0,03 | 0,02 | 0,02 |
| Coinitiator | 0,14 | 0,14 | 0,14 | 0,07 | 0,17 |
| PPD | 0,02 | 0,02 | 0,02 | 0,01 | 0,07 |
| Pigmente | 0,02 | 0,03 | 0,02 | 0,02 | |
| **Gesamt** | **100** | **100** | **100** | **100** | **100** |

**Tabelle II Messresultate**

| Material | Charge | Elastizitätsmodul in Mpa | | TCD-Monomer | Schrumpfspannung in Mpa¹ | | 3-Punkt Biegefestigkeit in Mpa | | **Quotient** | Std.abw. Quotient |
|---|---|---|---|---|---|---|---|---|---|---|
| | | E-Modul | Std.- abw. | | 24 h MW | Std.- abw. | Biegefestigkeit | Std.- abw. | | |
| Tetric Evo Ceram (Ivoclar Vivadent) | # G 20087 | 9172 | 308 | | 3,603 | 0,158 | 105,0 | 5,6 | **29,14** | 2,0 |
| Premise (Kerr) | #438811 | 7840 | 239 | | 3,679 | 0,143 | 93,0 | 5,5 | **25,28** | 1,8 |
| InTen-S (Ivoclar Vivadent) | # G 02023 | 9506 | 690 | | 3,822 | 0,181 | 109,0 | 11,9 | **28,52** | 3,4 |
| Filtek Supreme XT (3M Espe) | # 4 AP | 6528 | 374 | | 4,228 | 0,336 | 111,0 | 10,6 | **26,25** | 3,3 |
| EsthetX (Dentsply) | # 305000182 | 11606 | 585 | | 4,388 | 0,240 | 109,0 | 13,6 | **24,84** | 3,4 |
| Herculite XRV (Kerr) | # 06-1262 | 9585 | 305 | | 4,636 | 0,124 | 138,0 | 11,9 | **29,77** | 2,7 |
| Filtek Z250 (3M Espe) | # 4 LPJ | 12174 | 420 | | 4,887 | 0,289 | 153,0 | 15,3 | **31,31** | 3,6 |
| Spectrum TPH (Dentsply) | # 0612001961 | 10367 | 334 | | 4,900 | 0,250 | 127,0 | 10,5 | **25,92** | 2,5 |
| Xtra Fil (VOCO) | # 530835 | 16377 | 669 | | 4,912 | 0,212 | 132,0 | 8,0 | **26,87** | 2,0 |
| Quixfil (Dentsply) | # 0404000401 | 16257 | 783 | | 5,041 | 0,126 | 130,0 | 10,5 | **25,79** | 2,2 |
| Venus (Heraeus) | # 010119 | 9160 | 539 | | 5,180 | 0,252 | 120,0 | 9,6 | **23,17** | 2,2 |
| TPH³ (Dentsply) | # 0409171 | 9804 | 330 | | 5,515 | 0,167 | 128,0 | 13,0 | **23,21** | 2,5 |
| Grandio (VOCO) | # 441238 | 16498 | 544 | | 5,686 | 0,202 | 136,0 | 13,3 | **23,92** | 2,5 |
| | | | | | | | | | | |
| Vergleichsbeispiel 363 | | 13658 | 548 | 0 | 3,845 | 0,193 | 149,0 | 6,2 | **38,75** | 2,5 |
| Beispiel 312 | | 15767 | 560 | 1,61 | 3,250 | 0,087 | 130,0 | 10,4 | **40,00** | 3,4 |
| Beispiel 357 | | 13727 | 272 | 1,61 | 4,060 | 0,185 | 145,0 | 9,4 | **35,71** | 2,8 |
| Beispiel 349 | | 14262 | 661 | 10,5 | 3,330 | 0,143 | 171,0 | 7,6 | **51,35** | 3,2 |
| Beispiel 307 | | 13836 | 272 | 12,35 | 3,269 | 0,189 | 172,0 | 11,0 | **52,62** | 4,5 |

| | | | | | | | | | | |
|---|---|---|---|---|---|---|---|---|---|---|
| ¹ gemessen nach der Bonded-Disc-Methode - Dental Materials (2004) 20, 88-95) | | | | | | | | | | |

## Patentansprüche

1. Dental-Kompositmaterialien mit einem Gesamtfüllstoffgehalt von 70 bis 95 Gew.% enthaltend
A) in der Füllstoffkomponente 0,5 bis 10 Gew.% nicht-agglomerierte Nanofüller mit Partikelgrößen von 1 bis 50 nm;
B) in der Füllstoffkomponente mindestens 60 Gew.% eines Füllstoffgemischs aus 50 bis 90 % grob- und 10 bis 50 % feinteiligen Dentalgläsern, welche ein Größenverhältnis, bezogen auf die mittlere Partikelgröße (d₅₀-Wert), von feinteilig zu grobteilig von 1:4 bis 1:30 aufweisen,
i. in der als Monomermischung ein Anteil an TCD-Monomeren bezogen auf die Gesamtzusammensetzung von über 10 bis 15 Gew.%.
ii. in der als Monomermischung 10 bis 18 % UDMA bezogen auf die Monomermischung,
iii. in der als Monomermischung Rest TEDMA und/oder multifunktionelle Vernetzer
D) bis 1 % Initiator(en) und
E) optional in der Füllstoffkomponente mindestens ein weiteres Dentalglas mit sich von den grob- und feinteiligen Dentalgläsern unterscheidender Partikelgröße.
**dadurch gekennzeichnet, dass** der Anteil an TCD-Monomeren in der Gesamtzusammensetzung 10-15 Gew.% beträgt, und dass der Quotient Biegefestigkeit/Schrumpfspannung >/= 50 beträgt.

2. Dental-Kompositmaterialien nach Anspruch 1, **dadurch gekennzeichnet, dass** der Quotient Biegefestigkeit/Schrumpfspannung >/= 40 beträgt.

3. Dental-Kompositmaterialien nach Anspruch 1, **dadurch gekennzeichnet, dass** der Quotient Biegefestigkeit/Schrumpfspannung >/= 50 beträgt.
